Europäisches Patentamt

European Patent Office

Office européen des brevets

(11) Publication number: **0 094 116**
A2

# EUROPEAN PATENT APPLICATION

(21) Application number: 83200601.9

(22) Date of filing: 27.04.83

(51) Int. Cl.³: **A 61 K 9/54,** A 61 K 9/16

(30) Priority: 06.05.82 US 375428

(71) Applicant: **THE PROCTER & GAMBLE COMPANY, 301 East Sixth Street, Cincinnati Ohio 45201 (US)**

(43) Date of publication of application: **16.11.83 Bulletin 83/46**

(72) Inventor: **Close, Kenneth Stephen, 878 Yorkhaven Road, Springdale Ohio 45240 (US)**
Inventor: **Keim, Richard Edward, 12130 Brookston Drive, Springdale Ohio 45240 (US)**

(84) Designated Contracting States: **AT BE CH DE FR GB IT LI NL SE**

(74) Representative: **Suslic, Lydia et al, Procter & Gamble European Technical Center Temselaan 100, B-1820 Strombeek-Bever (BE)**

(54) **Therapeutic granules.**

(57) Superior time release therapeutic granules are achieved by applying a first coating of a therapeutic agent containing a dispersing aid to a therapeutic core and then applying a final enteric coating.

- 7 -

## Kenneth S. Close
## Richard E. Keim

### TECHNICAL FIELD

The present invention is related to therapeutic granules which have an improved rate of dissolution in intestinal fluid.

Analgesics such as aspirin are among the most widely used drugs of modern times and have been formulated in many different ways. One major concern faced with aspirin type analgesics is irritation to the stomach lining. To alleviate this formulators have provided buffers with the aspirin. The buffers help to provide a less acidic environment in the areas where the aspirin is in contact with the stomach. Another approach utilized in the prior art involves an enteric coating over the aspirin core. This approach utilizes a material which is resistant to the acidic environment of the stomach but is capable of dissolving (disintegrating) in the more alkaline intestinal environment. In spite of these efforts the need to provide safe and effective analgesic products still exists.

### BACKGROUND OF THE INVENTION

Specific disclosures concerning enteric coatings and buffers are contained in many different patents. U.S. Patent 3,524,910, August 18, 1970 to Holliday et al discloses analgesic compositions wherein the analgesic (e.g. aspirin) is coated with ethyl cellulose in a weight amount relative to the amount of analgesic of from 1:22 to 1:50. U.S. Patent 3,656,997, April 18, 1972 to Cordes discloses analgesic containing gelatin capsules having a first coating of a water/organic solvent soluble material and a second coating of an enteric material. U.S. Patent 3,691,090, September 12, 1972 to Kitajima et al discloses a method of encapsulating aspirin cores with an enteric polymer. U.S. Patent 3,906,086, September 16, 1975 to Guy et al discloses particles having an aspirin core and an enteric phthalate coating. U.S. Patent 4,308,251, December 29, 1981 to Dunn et al discloses aspirin tablets containing aspirin, an enteric material and an erosion promoting agent such as corn starch.

With respect to the use of buffers in aspirin containing products, U.S. Patent 3,954,959, May 4, 1976 to Pederson discloses buffers admixed with aspirin to form a core which then has a porous acid resistant coating applied. A similar patent is U.S. Patent 4,139,985, March 18, 1980 to Bechgaard et al. U.S Patent 3,131,123, April 28, 1964 to Masquelier discloses enteric coated granules of an analgesic-sodium bicarbonate mixture. U.S. Patent 3,922,339, November 25, 1975 to Shear discloses enteric coated medicants having buffers mixed in. U.S. Patent 3,341,416, September 12, 1967 to Anderson et al discloses ethyl cellulose coated aspirin particles, which particles have a buffer as a first coat.

While these references disclose the use of enteric coatings and buffers with analgesics, they do not disclose solutions to all of the problems associated with such drugs.

It has now been discovered that providing a disintegrating aid in a coating which surrounds the therapeutic core helps to provide improved release of the therapeutic agent in the intestines.

It is, therefore, an object of the present invention to provide an effective enteric coated therapeutic agent.

It is a further object of the present invention to provide enteric coated therapeutics having a dispersing aid present in a coating surrounding the therapeutic core, which coating has attached to it the enteric coating.

It is still a further object of the present invention to provide a safe and effective method of treating pain.

These and other objects will become readily apparent from the detailed disclosure which follows.

All percentages and ratios herein are by weight unless otherwise specified.

## DESCRIPTION OF THE INVENTION

The present invention relates to enteric therapeutic granules comprising:

(a) a non-steroidal, therapeutic active core;

(b) a first coating adhered to said core of a non-steroidal, therapeutic active;

(c)  a dispersing aid contained within said first coating; and

(d)  a second coating adhered to said first coating of an enteric material;

wherein said enteric coated granules are from about 0.5mm to about 1.5mm in diameter, preferably from about 0.7mm to about 1.2mm, the weight ratio of said therapeutic core to said first coating is from about 1:0.3 to about 1:1, the amount of dispersing aid contained within said first coating is from about 2% to about 15% of said coating; and said enteric coating is from about 2.0% to about 10.0% of the entire enteric coated granule.

The essential as well as optional components of the invention claimed herein are set forth in the paragraphs which follow.  In the present application the following terms have the meanings given.

"Pharmaceutically-acceptable" or "pharmacologically-acceptable", as used herein, means that the ingredients used in the compositions are suitable for use in contact with the tissue of humans, without undue toxicity, irritation, allergic response, and the like, commensurate with a reasonable benefit/risk ratio.

The term "comprising", as used herein, means that various other compatible components, including both active and inert ingredients, can be conjointly employed in the compositions of this invention.  The term "comprising" thus encompasses and includes the more restrictive terms "consisting of" and "consisting essentially of".

By "compatible" herein is meant that the components of the present invention are capable of being commingled without interacting in a manner which would substantially decrease the efficacy of the therapeutic under ordinary use conditions.

All percentages and ratios of the components are set forth hereinbelow.

## Non-Steroidal Analgesic Active Core

The core of the granules described and claimed herein is a non-steroidal, therapeutic agent.

- 4 -                      0094116

The therapeutic actives used in the present invention preferably have analgesic, antipyretic and anti-inflammatory properties. Aspirin is an example of such a material and is the preferred material. However, there are numerous other materials which can be used. These include calcium carbaspirin, chloline salicylate, salicylic acid, naproxen, ibuprofen, fenoprofen, zomepirac and mixtures thereof.

The core of the present granules generally contains about 50% to about 100%, preferably from about 85% to about 95% of the therapeutic active.

Optional components which may be used in the cores include diluents, binders, disintegrents, lubricants, glidents, buffering adjuvants and direct acting adjuvants. Numerous pharmaceutically acceptable and compatible materials can be found in various pharmacological references. Two such references are Pharmaceutical Dosage Forms: Tablets, Vol. 1, Ed. H. A. Lieberman and L. Lachman, Marcel Dekker, Inc., 1980 and "Proposed Monograph for OTC Internal Analgesic, Antipyretic and Antirheumatic Drugs", Federal Register, July 8, 1977. Both are incorporated herein by reference.

Exemplary materials of the above types include dicalcium phosphate and lactose as diluents; gelatin and polyvinylpyrrolidone as binders; starch, sodium starch glycolate cellulosics, and cross-linked polyvinylpyrrolidone as disintegrents; stearates as lubricants; fumed silicas as glidents; glycine, carbonate salts, magnesium and aluminum hydroxides, aluminum glycinate and borate salts as buffering adjuvants; and caffeine, ascorbic acid and other vitamins, antihistamines and other cough, cold, allergy bronchodilator and antiasthamatic drugs as direct acting adjuvents.

These optional components individually may be present at a level of about 0% to about 50%, preferably from about 5% to about 15% of the core.

Active Coating

The active coat which is adhered to the core of the granules can be any of the actives mentioned previously as suitable for core use. Additionally, other materials such as acetaminophen can be used. It should also be recognized that the active coat may be a different drug than the core but is preferably the same. Aspirin is the preferred material.

The weight ratio of the core to the first (active) coat is from about 1:0.3 to about 1:1, preferably from about 1:0.5 to about 1:0.8. Amounts of coating within these ranges allow for adequate covering of the rough core surface while not making the total granule excessively large.

Included among optional components in the first coat, as set forth in the discussion of the core, are binders and direct acting adjuvents. An amount of said optional components, individually, found useful is from about 4% to about 50%, preferably from about 6% to about 20% by weight of the active coating.

Dispersing Aid

Included in the active coating is a dispersing material to aid in the disrupting of the enteric coating in the intestines. The dispersing aid affects the pH of its environment and includes materials such as carbonate salts, phosphate salts, magnesium hydroxide, aluminum hydroxide, borate salts, aluminum glycinate, amino acids such as alanine, aspartic acid, glutamic acid histidine, isoleucine, tyrosine, phenylalanine, cystine, leucine, methionine, proline, hydroxyproline, valine and tryphtophonine and mixtures thereof. The preferred agents are the alkali metal phosphate salts and glycine.

The amount of dispersing aid should be from about 2% to about 15% of the active coating, preferably from about 5% to about 10%.

Enteric Coating

The materials useful as the enteric coating for the present granules include any pharmaceutically acceptable material insoluble at pH's of from about 1 to about 3 while being soluble at pH's in the range of from about 4.5 to about 8.0. Suitable materials include hydroxypropyl methylcellulose phthalate, cellulose acetate phthalate, polyvinyl acetyl phthalate and acrylic anionic polymers. Various fats and oils and mixtures of the foregoing are also acceptable. The enteric coating is present at a level of 2.0% to about 10.0%, preferably from about 4% to about 6% of the entire enteric coated granule.

Optional components suitable for combining with the enteric material include surface active agents and plasticizers such as triethyl citrate, triacetin, triglycerides and acetylated monoglyceride.

## METHOD OF MANUFACTURE

The following procedure can be used to make granules of the type disclosed and claimed herein.

Commercially available (Dow Chemical Co.) aspirin starch granules (90% aspirin/10% starch) are screened to provide granules with a diameter range of 0.5 to 1.0mm. Said granules are then coated with the therapeutic coating using a Wurster Fluidized bed coater (Glatt Air Techniques, Inc., Model WSG5) with a seven inch column diameter.

Two thousand grams of sieved granules are placed in the Wurster coater. The therapeutic coat components (Example 1) are slurried with a 1:1.5 weight:volume ratio of isopropyl alcohol and maintained in a suspended state during the coating application via agitation. The coating slurry is applied at a rate of 140 ml/min. through a 1.0mm nozzle and atomized with 60 l/min. air at 90 psig. The bed is fluidized with 2180 l/min. air at 52°C. inlet temperature. The exit air temperature under these conditions is about 30°C. The coating operation is continued until 4000 ml (1500g solids) of therapeutic coating are applied.

The therapeutically coated granules are then sieved to a particle size range of 0.5 to 1.2mm and 1900g are placed in the Wurster coater. An 9.0% solution of the enteric coat components (Example 1) in an azeotrope mixture of ethyl acetate and isopropyl alcohol (75:25 wt.:wt.) is then prepared. The coating solution is applied at a rate of 40 ml/min. through a 0.8mm nozzle and atomized with 70 l/min. air at 90 psig. The bed is fluidized with 2180 l/min. air at 63°C. inlet temperature. The exit air temperature is about 40°C. under these conditions. The coating operation is continued until 1250 ml of solution (100g solids) are applied.

The enteric coated particles are then sieved to a size range of 0.5 to 1.2mm and formulated into the final dosage form.

<u>FIELD OF USE</u>

The granules of the present invention can be used in tablets, capsules or in any other convenient form. The therapeutic agents are well recognized for treating a wide variety of human ailments.

The following non-limiting examples illustrate the compositions of the present invention.

- 8 -

### EXAMPLES I - IV

| Core | I (53.7%) | II (51.1%) | III (52.9%) | IV (50.0%) | % of total granule |
|---|---|---|---|---|---|
| Aspirin | 90.0 | 94.0 | 95.0 | 90.0 | |
| Starch | 10.0 | | | 10.0 | |
| Crosslinked carboxy-methyl cellulose | | | 3.0 | | |
| Sodium starch glycolate | | 6.0 | | | |
| Polyvinyl pyrrollidone | | | 2.0 | | |
| | 100.00 | 100.00 | 100.00 | 100.00 | |
| Active Coat | (40.3%) | (40.9%) | (37.1%) | (45.0%) | % of total granule |
| Aspirin | 85.0 | 85.0 | 85.0 | 80.0 | |
| Polyvinyl-pyrrollidone | 4.0 | 4.0 | 5.0 | | |
| Sodium starch glycolate | 2.0 | 2.0 | 2.0 | | |
| Starch | | | | 10.0 | |
| Glycine | | 9.0 | | | |
| Histidine | | | 8.0 | | |
| $Na_2HPO_4$ | 9.0 | | | | |
| $Mg(OH)_2$ | | | | 10.0 | |
| | 100.00 | 100.00 | 100.00 | 100.00 | |

EXAMPLES I – IV  (continued)

| Enteric Coat | I (6.0%) | II (8.0%) | III (10.0%) | IV (5.0%) | % of total granule |
|---|---|---|---|---|---|
| Hydroxypropyl methyl-cellulose phthalate | 90.0 | 75.0 | 90.0 | | |
| Cellulose acetate phthalate | | | | 90.0 | |
| Acetylated mono-glycerides | 10.0 | | 10.0 | | |
| Triethyl citrate | | 25.0 | | 10.0 | |
| | 100.00 | 100.00 | 100.00 | 100.00 | |

The above compositions are capable of reducing aspirin contact with the acidic environment of the stomach while still providing satisfactory release of the aspirin in the intestines.

CLAIMS

1.  Therapeutic, enteric coated granules characterized in that they comprise:

    (a) a non-steroidal, therapeutic active core;

    (b) a first coating adhered to said core of a non-steroidal, therapeutic active;

    (c) contained within said first coating a dispersing aid; and

    (d) a second coating adhered to said first coating of an enteric material;

    wherein said enteric coated granules are from 0.5mm to 1.5mm in diameter, the weight ratio of said core to said first coating is from 1:0.3 to 1:1, said dispersing aid comprises from 2% to 15% by weight of said first coating and said enteric coating is from 2% to 10% by weight of the entire coated granules.

2.  Enteric coated granules according to Claim 1 characterized in that the therapeutic active core is selected from the group consisting of aspirin, calcium carbaspirin, choline salicylate, magnesium salicylate, sodium salicylate, salicylic acid, naproxen, ibuprofen, fenoprofen, zomepirac and mixtures thereof.

3.  Enteric coated granules according to Claim 2 characterized in that the first coating is selected from the group consisting of aspirin, calcium carbaspirin, chloline salicylate, magnesium salicylate, sodium salicylate, salicylic acid, naproxen, ibuprofen, fenoprofen, zomepirac, acetaminophen, and mixtures thereof.

4.  Enteric coated granules according to Claim 3 characterized in that the enteric coating is selected from the group consisting of hydroxypropyl methylcellulose phthalate, cellulose acetate phthalate, polyvinyl acetyl phthalate, acrylic anionic polymers, fats, oils and mixtures thereof.

5.  Enteric coated granules according to Claim 4 characterized in that the dispersing aid is selected from the group consisting of carbonate salts, phosphate salts, magnesium hydroxide, aluminum hydroxide, borate salts, aluminum glycinate, amino acids such as alanine, aspartic acid, glutamic acid, histidine, isoleucine, tyrosine, phenylalanine, cystine, leucine, methionine, proline, hydroxyproline, valine and tryphtophonine and mixtures thereof.

6. Enteric coated granules according to Claim 5 characterized in that the size of said granules is from 0.7mm to 1.2mm.

7. Enteric coated granules according to Claim 1 characterized in that the first coating is selected from the group consisting of aspirin, acetaminophen and mixtures thereof.

8. Enteric coated granules according to Claim 7 characterized in that the dispersing aid is selected from the group consisting of alkali metal phosphates and glycine.

9. Enteric coated granules according to Claim 8 characterized in that the enteric material is selected from hydroxypropyl methylcellulose phthalate, polyvinyl acetyl phthalate and mixtures thereof.

10. Enteric coated granules according to Claim 9 characterized in that the therapeutic active core is aspirin, said first coating is aspirin, said dispersing aid is an alkali metal phospahte and said enteric coating is hydroxypropyl methylcellulose phthalate.